# DEMANDE DE BREVET EUROPEEN

(11) **EP 0 538 201 A1**
(43) Date de publication de la demande: **21.04.1993**
(21) Numéro de dépôt: 92830465.8
(22) Date de dépôt: 02.09.1992
(51) Int. Cl.: A61F 2/60

(54) **Prothèse pour membre inférieur perfectionnée**

(30) Priorité: 04.09.1991 IT FI910217
(71) Demandeur: TOSCOFARMA ELECTROBIOMEDICAL S.A.S., I-51020 Cireglio (Pistoia) (IT)
(72) Inventeur: TOSCOFARMA ELECTROBIOMEDICAL S.A.S., I-51020 Cireglio (Pistoia) (IT)
(74) Mandataire: Martini, Lazzaro

(57) **Abrégé**

Prothèse pour membre inférieur perfectionnée dans laquelle l'articulation du genou est réalisée avec deux éléments (4,5) en forme de fourche, avec les branches repliées en arrière et articulées entre elles en un point décalé en arrière par rapport à l'axe vertical yy; et l'articulation du pied est réalisée avec deux éléments (7,70) articulés sur l'axe yy, le premier (7) étant muni d'un appui arrière situé au-dessous (74) solidaire du pied (10) et d'un piston (9) en forme de pipe retournée, situé au-dessous, coulissant dans un tube (8) solidaire de l'élément (7) et dont l'extrémité (91) forme l'appui antérieur sur une saillie (10) de l'élément inférieur (70).
L'articulation du genou est fixée sur la cuisse du porteur avec un plateau (1) dont le point A décalé en avant par rapport à l'axe yy et dont l'ergot inférieur (20) est logé dans un siège central de la tête (3).
Les deux articulations sont reliées par une biellette (12) articulée à son extrémité supérieure avec la fourche (5) en un point C qui est décalé en avant par rapport au point B, et à son extrémité inférieure avec un tirant (14,15) fixé au piston (9) et sur lequel est enfilé un ressort (17) de poussée.

## Description

La présente invention concerne une prothèse pour membre inférieur perfectionnée.

Il est connu d'après le document EP n^{o} 41.052 du même titulaire, une prothèse pour membre inférieur comprenant deux articulations, une au niveau du genou et l'autre au niveau du pied, lesquelles sont reliées aux extrémités, supérieure et respectivement inférieure, d'un montant tubulaire dans lequel sont logés un tirant et un piston de connexion avec ressort de poussée.
L'articulation du genou est constituée de deux leviers superposés, pivotant autour d'axes parallèles, horizontaux, décalés en arrière par rapport à l'axe vertical de la prothèse et munis de dentures qui engrènent l'une avec l'autre. L'articulation du pied est consituée de deux éléments superposés à charnière horizontale et de deux ressorts disposés de part et d'autre et exerçant des force antagonistes par rapport à la charnière, un de ces ressorts étant supporté par une tige extérieure au montant, associée au piston précité.

Cette prothèse connue permet d'obtenir, pendant la déambulation, le redressement du genou pour l'avancement spontané de la jambe lorsque le pied est soulevé du sol et la rotation contrôlée du pied lorsqu'il supporte le poids du porteur pendant l'avancement de l'autre jambe, mais présente certains inconvénients tels que la complexité de construction qui entraîne des dimensions volumétriques excessives du genou et de la malléole en empêchant un revêtement esthétiquement acceptable et moderne de la prothèse.

La présente invention a pour but d'éliminer ces inconvénients et, en outre, d'assurer la stabilité maximale avec des moyens de fixation à la cuisse du porteur.

Ces résultats ont été atteints conformément à l'invention en réalisant:
- l'articulation du genou avec deux éléments en forme de fourche, avec les branches repliées en arrière et articulées entre elles en un point décalé en arrière par rapport à l'axe vertical;
- l'articulation du pied avec deux éléments articulés sur l'axe de la structure squelettique, le premier étant muni d'un appui arrière, situé au-dessous, solidaire du pied, avec ressort, et d'un piston placé au-dessus et dont le pied forme l'appui antérieur sur une saillie de l'élément inférieur: l'articulation du genou étant fixée sur la cuisse du porteur en un point A décalé en avant par rapport à l'axe vertical et avec un ergot inférieur logé dans un siège central de la tête de la prothèse et les deux articulations étant reliées dans la partie supérieure par une biellette articulée en un point décalé en avant par rapport à la charnière du genou et dans la partie inférieure à un tirant fixé au piston de la charnière du pied et sur lequel est enfilé un ressort de poussée.

Les avantages obtenus grâce à l'invention consistent essentiellement en ce qu'il est possible de réaliser une prothèse très légère, d'application facile, d'utilisation pratique et sûre, de fiabilité très élevée même après un longue période de service.

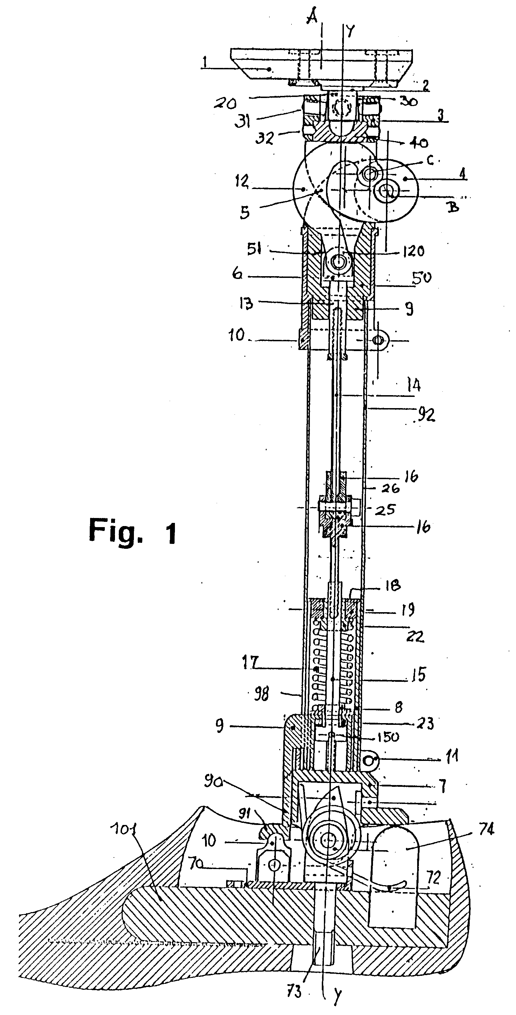

Ces avantages et caractéristiques de l'invention ainsi que d'autres seront plus et mieux compris de chaque homme du métier à la lumière de la description qui va suivre et à l'aide du dessin annexé donné à titre d'exemplification pratique de l'invention, mais à ne pas considérer dans le sens limitatif; dessin sur lequel l'unique Figure représente une vue en coupe verticale axiale d'une prothèse conformément à l'invention.

Réduite à sa structure essentielle et en référence au dessin annexé, une prothèse pour membre inférieur perfectionée, conformément à l'invention, est constituée par:
A) Des moyens d'application sur la cuisse avec un plateau 1 horizontal à fixer sur le point central A de la cuisse du porteur située au-dessus (non représenté par esprit de simplification), ce point se trouvant décalé de 10 mm dans la direction de la pointe du pied, par rapport à l'axe vertical yy de la structure squelettique.
   Ledit plateau 1 est fixé par le bas au sommet de la structure squelettique au moyen d'une fixation avec ergot 20 centré sur l'axe YY et logé dans une cavité correspondante 30 d'une tête 3 de la prothèse et fixé à cet endroit au moyen de plusieurs vis sans tête à six pans creux horizontales 31 qui permettent le réglage vertical de la cuisse.
B) Une articulation au niveau du genou comprenant:
   - un élément supérieur en forme de fourche 4 avec les branches recourbées en bas vers l'arrière, c'est-à-dire du côté du talon du pied, et avec la base cylindrique 40 logée pivotante autour de l'axe YY dans un logement correspondant de ladite tête 3 pour permettre son orientation horizontale et d'être fixée à cet endroit au moyen de vis sans tête à six pans creux 32;
   - un élément inférieur en forme de fourche 5 avec les branches recourbées en haut vers l'arrière et articulées en B aux branches de la fourche supérieure 4 et avec la base cylindrique 50 enfilée axialement dans un manchon vertical 6.
C) Une articulation au niveau du pied comprenant:
   - un élément supérieur 7, monté oscillant sur le pivot 71 de l'élément inférieur 70 avec un appui élastique arrière 74, auquel est fixé un tube vertical 8 avec fente 80 longitudinale antérieure, dans laquelle est logé coulissant un piston en forme de pipe retournée 9 avec la tige 90 parallèle à l'extérieur du tube 8: l'extrémité inférieure 91 de la tige 90 ayant un profit actif curviligne avec la concavité orientée vers te bas, laquelle est destinée à coopérer avec la partie dorsale curviligne d'une saillie antérieure 10 située au-dessous et réalisée en darlin. Ledit piston est avantageusement revêtu d'une protection en téflon;
   - un élément inférieur 70 logé dans une cavité 102 du pied 101 auquel il est fixé au moyen d'une vis 73 et lequel supporte te pivot précité 71 avec un ressort 72 et ladite saillie 10.
D) Des moyens de connexion entre les deux articulations du genou et du pied, avec érection lors du ploiement du genou et inversement, comprenant:
   - un montant 92 tubulaire avec une fente longitudinale antérieure 93 juxtaposée avec celle 80 du tube 8, ce montant étant fixé à son extrémité supérieure à l'élément 6 de l'articulation du genou au moyen d'un étau 10 et à son extrémité inférieure à l'élément 7 de l'articulation du pied au moyen d'un étau 11;
   - une biellette 12 en forme de crochet, dont la boucle est articulée de manière excentrique en C avec l'élément inférieur 5 de l'articulation du genou et dont le pied 120, qui est logé dans une cavité axiale 51 de la base 50 précitée, est articulé avec une fourche 13 située au-dessous;
   - une tige verticale 14, fixée à son extrémité supérieure au pied de ladite fourche 13 et à son extrémité inférieure à un tirant 15 avec interposition d'un accouplement 16 à baïonnette; ledit tirant passe à travers un trou axial correspondant du piston 9 précité et du tube 8 précité de l'articulation du pied, et sur ce tirant est enfilé un ressort hélicoïdal de poussée 17, qui est en appui sur un diaphragme 18 avec interposition d'une cale 19 avec un joint en téflon 22. Un joint amortisseur 23 est prévu entre la base 150 du tirant 15 et le piston 9, lequel doit être monté avant de passer le tirant 15 dans le piston 9 et d'appliquer la goupille 24; après quoi, on monte le ressort 17 sur le tirant 15 avec un joint en téflon. Pour unir la tige 14 au tirant 15, il suffit d'enclencher l'accouplement 16 et de bloquer la vis 25 à travers la fente 26 du montant 92 après que celui-ci a été fixé à l'élément 7 au moyen de l'étau 11.

Avantageusement, ledit point C de la biellette 12 est prévu en correspondance d'un carré de 8,5 mm de côté et dont une des diagonales unit ledit point C avec le centre de rotation de l'articulation 4,5 du genou.
De plus, conformément à l'invention, la compression du ressort 17 est réglable au moyen d'une ou plusieurs rondelles à interposer entre la cale 19 et le joint 22.

## Revendications

1. Prothèse pour membre inférieur comprenant une articulation (4,5) du genou et une articulation (7,70) du pied disposées aux extrémités d'un montant vertical (15), des moyens (1) pour la fixation à la cuisse du porteur, des moyens de connexion entre tes deux articulations pour obtenir l'érection du pied lors du ploiement du genou, caractérisée en ce que ladite articulation du genou est composée par:
- un élément supérieur en forme de fourche (4) avec rapport à la pointe du pied et avec une base cylindrique (40) pivotant autour de l'axe vertical YY;
- un élément inférieur en forme de fourche (5), avec les branches recourbées en haut vers l'arrière, lesquelles sont articulées en B aux branches de la élément supérieur (4) en position décalée vers l'arrière par rapport à l'axe YY, et avec une base cylindrique (50) tournant autour de l'axe YY, à l'intérieur d'un manchon (6);
en ce que ladite articulation du pied est composée par:
- un élément supérieur (7) articulé sur le pivot (71) et avec un appui élastique arrière (74);
- un tube (8) situé au-dessus avec fente (80) longitudinale antérieure;
- un piston en forme de pipe retournée (9) avec jupe coulissant dans ledit tube (8) et dont la tige (90) s'étend verticalement à l'extérieur vers le bas avec une partie en porte-à-faux (91) curviligne avec concavité orientée vers le bas:
- un élément inférieur (70) fixé au pied (101) dans une cavité (100), sur lequel sont montés le pivot (71) avec un ressort (72) et une saillie (10) d'appui pour la partie en porte-à-faux (91) du piston (9);
en ce que lesdits moyens de fixation à la cuisse du porteur sont constitués par:
- un plateau horizontal (1) fixé centralement sur la cuisse située au-dessus en un point A décalé en avant par rapport à l'axe vertical YY et muni d'un ergot inférieur (20) centré sur l'axe YY, à fixer dans une cavité correspondante (30) d'une tête (3) située au-dessous;
et en ce que lesdits moyens de connexion entre les deux articulations du genou et du pied, pour obtenir l'érection et respectivement la détente du pied lors du ploiement et respectivement de la détente du genou, sont constitués par:
- une biellette (12) en forme de crochet, dont la boucle est articulée de manière excentrique en C avec l'élément inférieur (5) de l'articulation du genou et dont le pied (120) est articulé avec une fourche (13) située au-dessous;
- un tirant (15) ancré à l'extrémité inférieure au piston précité (91) et prolongé à son extrémité supérieure et rendu solidaire de ladite fourche (13) au moyen d'une tige (14) et avec interposition d'un accouplement 16 à baïonnette;
- un ressort hélicoïdal de poussée (17), enfilé sur ledit tirant (15) et comprimé entre la jupe (89) du piston (9) et un diaphragme (18) du montant (92).

2. Prothèse selon la revendication 1, caractérisée en ce que le centre fixation dudit plateau (1) à la cuisse du porteur de la prothèse est décalée en avant de 10 mm par rapport à l'axe vertical YY de la structure squelettique.

3. Prothèse selon la revendication 1, caractérisée en ce que le point de rotation B des éléments (4,5) de l'articulation du genou est décalé en arrière de 22 mm par rapport à l'axe vertical YY de la structure squelettique.

4. Prothèse selon la revendication 1, caractérisée en ce que le point de rotation C de la biellette (12) par rapport à l'élément inférieur (51) de l'articulation du genou se trouve en correspondance d'un sommet d'un carré de 8,5 mm de côté et dont une diagonale unit ledit sommet avec le point de rotation B de l'articulation du genou.

5. Prothèse selon la revendication 1, caractérisée en ce que la saillie (10) de l'élément (70) est réalisée en darlin.

6. Prothèse selon la revendication 1, caractérisée en ce que ledit montant (92) est muni d'une fente (26) pour permettre l'introduction et le blocage d'une vis (25) de connexion de l'accouplement (16).

7. Prothèse selon la revendication 1, caractérisée en ce que ledit tirant (15) est muni d'une rondelle (23) interposée entre la base (150) du tirant (15) et le piston (9).

8. Prothèse selon la revendication 1, caractérisée en ce que ledit tirant (15) est muni d'une cale (19) avec joint (22) en téflon, interposés entre ledit ressort (17) et ledit diaphragme (18).

9. Prothèse selon la revendication 1, caractérisée en ce qu'elle comprend une ou plusieurs rondelles interposées entre la cale (19) et le joint (22) pour régler la compression du ressort (17).
